# EUROPÄISCHE PATENTSCHRIFT

(11) **EP 0 355 125 B1**
(45) Veröffentlichungstag und Bekanntmachung des Hinweises auf die Patenterteilung: **22.09.1993**
(21) Anmeldenummer: 88907077.7
(22) Anmeldetag: 09.06.1988
(51) Int. Cl.: G01N 33/574, A61K 39/00

(54) **VERFAHREN ZUR INTEGRIERTEN ONKOLOGISCHEN EINGANGSUNTERSUCHUNG UND KREBSTHERAPIE**
PROCESS FOR INTEGRATED ONCOLOGICAL ADMISSION EXAMINATION AND CANCER THERAPY
PROCEDE D'EXAMEN ONCOLOGIQUE D'ADMISSION ET DE THERAPIE ANTI-CANCEREUSE INTEGRES

(30) Priorität: 12.06.1987 DE 3719698
(43) Veröffentlichungstag der Anmeldung: 28.02.1990
(73) Patentinhaber: Bartos Patent Development and Holding Company Limited, Dublin 2 (IE); BARTOS, Stefan, Dr., D-42697 Solingen (DE)
(72) Erfinder: BARTOS, Stefan, D-5650 Solingen 1 (DE)
(74) Vertreter: Werner, Hans-Karsten, Dr.Dipl.-Chem.
(86) Internationale Anmeldenummer: EP8800509
(87) Internationale Veröffentlichungsnummer: WO8809935

(56) Entgegenhaltungen:
- EP-A- 0 092 223
- Science, vol. 235, n 4785, 9 January 1987 (Washington DC, US), D.J. Slamon et al "Human breast cancer : correlation of relapse and survival with amplification of the HER-2/neu oncongene", see pages 177-182

## Beschreibung

Gegenstand der vorliegenden Erfindung ist ein Verfahren zur integrierten onkologischen Eingangsuntersuchung der autologen Tumorantigene und Krebstherapie durch Impfung oder Vakzination mit aus Tumorzellen stammendem Material sowie autologes Tumorantigen in lösbarer und in nicht lösbarer Fraktion für immunbiologische Untersuchungen, für Hauttest- und für Impf-/Vakzinationszwecke.

In der Krebstherapie ist neben dem operativen Vorgehen - je nach Tumorform - die zusätzliche Bestrahlung, bzw. die Anwendung von zytostatischen Substanzen, die sogenannte Chemotherapie, sowie ggf. die Hormontherapie etabliert. Obwohl die Erkenntnisse über die immunbiologische Auseinandersetzung des Tumorträgers (Patient) mit seinem Tumor (Krebszellen) von Tag zu Tag zunehmend sind, steht man bei der Erschließung der Tumorimmunologie als therapeutische Reserve erst am Anfang. Diese Aufgabe stellt eine wissenschaftliche Herausforderung dar.

Die bisherigen Bemühungen, die Tumorimmunologie einer praktischen Anwendung zuzuführen, lassen sich grundsätzlich in zwei verschiedene methodische Richtungen einordnen.

Eine Grupppe bemüht sich, die Art und Weise der immunbiologischen Auseinandersetzung des Wirtes mit dem Tumorgeschehen mit verschiedenen Untersuchungstechniken passiv zu analysieren, um feststellen zu können, welche prognostische Bedeutung diesen Reaktionen zuzuordnen ist. Hierbei handelt es sich meistens um ex-vivo-invitro-Untersuchungstechniken. Dieses Vorgehen hat viele positive Ergebnisse erbracht, die nachstehend am Beispiel von Brust- und Eierstockkrebs näher erläutert werden.

Eine weitere nicht zu vernachlässigende Zahl von Forschern hat sofort eine medikamentöse Therapie verordnet, in der Erwartung, hiermit den Krankheitsverlauf positiv zu beeinflussen. So ist aus klinischer Sicht der alleinige Einsatz von BCG-Vakzination bei verschiedenen Krebsformen ein allgemeinbekannter historischer Versuch. Partiell mehr positive Resultate haben Autoren erzielt, die z.B. bei Dickdarmkrebspatienten mit einer homologen etablierten Dickdarmkrebszellinie vakziniert, bzw. geimpft haben. Ähnliche Erfahrungen wurden bei Prostatakrebspatienten gemacht. Noch bessere klinische Resultate wurden erzielt, wenn man die homologen Impfzellen vorher z.B. mit Neuraminidase behandelt hatte. Die homologen Tumorzellen wurden vor der Impfung in geeigneter Weise, z.B. durch subletale Bestrahlung vermehrungsunfähig gemacht. Bei dieser Art der Behandlung, die als unkontrolliert zu bezeichnen ist, waren jedoch eklatante Fehlschläge zu verzeichnen. Statt Effektivität oder zumindest einer Unwirksamkeit wurde wiederholt ein beschleunigtes Tumorwachstum, ein sogenanntes Enhancement, induziert. Diese Vorgehensweise konnte sich nicht etablieren.

Einen weiteren Fortschritt brachte die immuntherapeutische Technik, wobei vitalisolierte, autologe Tumorzellen mit geeigneten Maßnahmen teilungsunfähig gemacht wurden, z.B. durch in-vitro Mitomycin-C-Behandlung, und diese Tumorzellen alleine oder kombiniert mit BCG oder nach Neuraminidase-Behandlung zur Vakzination, vorzugsweise intralymphatisch appliziert, eingesetzt wurden.

Zur Vermeidung eines negativen Effektes, einer Beschleunigung des Tumorwachstums (Enhancement), wurde zur optimalen Dosisfindung die Schachbrett-Titration eingeführt. Hierbei werden Tumorantigendosen, vorzugsweise in der Haut des Rückens des Patienten, in steigenden Dosen, erfahrungsgemäß in der Größenordnung von 10⁵ bis 10⁷, max. 10⁸ Tumorzellen pro Impfstelle appliziert. Als Zeichen einer Abwehrreaktion tritt im positiven Falle eine Rötung um die Applikationsstelle der optimalen Antigendosis/Antigendosen auf (positive Hautreaktion vom verzögerten-Tuberkulin-Typ). Im weiteren Vorgehen richtet man sich dann nach dieser Hautreaktion. Insbesondere ein Negativwerden der Reaktion zeigt die Notwendigkeit einer erneuten Vakzination an.

In den letzten Jahren kam die Erkenntnis, daß im Rahmen einer tumorassoziierten Immunreaktion ein ganzes Spektrum von Antigenen, wie z.B. Differenzierungsantigene, organspezifische, onkofetale und vor allem individualspezifische Antigene reagieren und das Vorhandensein, d.h. die Expression dieses Antigenspektrums von Patient zu Patient und sogar von Tochtergeschwulst zu Tochtergeschwulst in erheblichem Umfange variiert. Dies ist die Begründung dafür, weshalb man mit autologen Tumorantigenpräparationen bessere Resultate erzielen kann, so passiv im Rahmen der Krebstherapie begleitenden Diagnostik, wie beim aktiven therapeutischen Vorgehen, d.h. bei Vakzination.

Auch wenn viele der bisher durchgeführten immuntherapeutischen, immunmodulierenden klinischen Studien positive Resultate aufweisen konnten, waren diese Studien fast ausnahmslos mit dem Fehler behaftet, daß bei einer Tumorform, z.B. beim Brustkrebs, einfach angenommen wurde, daß bei jeder Mammakarzinompatientin eine einheitliche tumorassoziierte Abwehrreaktion gegeben sei, die einheitlich beeinflußt werden könnte. Es wurde übersehen, bzw. schlicht ignoriert, daß für positive immuntherapeutische Ansätze die Voraussetzung erfüllt sein muß, daß nur mit einem geeigneten Präparat, an einem dafür geeigneten Objekt zu einem dafür geeigneten Zeitraum therapiert werden darf. Ansonsten ist ein Fehlschlag vorprogrammiert.

Zwar hat die tumorassoziierte Immunreaktion kein Privileg. Ganz vereinfacht kann gesagt werden, daß der erste Schritt bei der Auseinandersetzung des Tumorträgers mit den Krebszellen ebenfalls auf der Ebene der Makrophagen und der Natural-Killer-Zellen und dann im Zusammenwirken mit den funktionell verschiedenen Subpopulationen der T-Zellen stattfindet. Erst danach tritt eine B-Zell-Immunantwort auf, mit dem Erscheinen und der Nachweisbarkeit von tumorassoziierten Antikörpern, in der Regel mit den Immunglobulin-Typen Ig-M, Ig-A und Ig-G.

Die tumorassoziierten Antikörper können wiederum die zelluläre Zytotoxizität gegenüber den Tumorzellen verstärken. Jedem Experten ist es bekannt, daß die höchste Effizienz einer antikörpergesteuerten, komplementabhängigen, zellulären Zytotoxizität zuzuordnen ist, wo Antikörper des Typs Ig-M und/oder Ig-G nachweisbar sind.

Erst die Klärung der Qualität und der Stärke einer tumorassoziierten zellulären und humoralen Immunität gibt endgültig darüber Aufschluß, welches zusätzliche prognostische Kriterium die tumorassoziierte Immunreaktion darstellt und welches zusätzliche therapeutische Potential diese beinhaltet.

So wird in der Krebstherapie unverweigerlich die Forderung nach routinemäßig durchführbaren immunbiologischen Eingang- und Kontrolluntersuchungen gestellt. Eine blinde, unkontrollierte spezifische Immuntherapie oder eine einfache unspezifische Reiztherapie mit immunmodulierenden Substanzen ist strikt abzulehnen und wird aus mehreren Gründen als gefährlich bezeichnet. Jede biologische Therapie ist eine zweischneidige Waffe, die sowohl gewünschte als auch ungewünschte Effekte bei dem gleichen Patienten bewirken kann.

Den Idealzustand stellt die Situation dar, daß für jeden Krebspatienten eigene, autologe Tumorzellen isoliert werden und diese entweder in Gewebekultur gehalten oder vital im flüssigen Stickstoff eingefroren werden, um bei Bedarf auftauen zu können. Diese vitalen, patienteneigenen Tumorzellen stellen dann laufend die geeigneten Zielzellen für immunbiologische Kontrolluntersuchungen dar und können gleichzeitig zur Immunisierung/Vakzination des Patienten benutzt werden. Führt man die geeignetesten Untersuchungen, wie z.B. Cr-Re-leasing-Test, Koloniebildung-Hemmtest, usw. vor und nach immunrestaurativen-, immunmodulierenden Maßnahmen oder nach Vakzination durch, so kann exakt die Wirkung und der Sinn oder Unsinn der therapeutischen Maßnahmen beurteilt werden.

Dieses Vorgehen kann in der Routine aus verschiedenen, vor allem aus histopathologischen und technischen Gründen, nur in Ausnahmefällen, meist für Forschungsfälle sichergestellt werden und bleibt auf die Dauer mangels entsprechender Ressourcen nicht bezahlbar. So bedarf es der Entwicklung und Etablierung äquivalenter Techniken, die rationell durchführbar sind.

Es wurde gefunden, daß die oben aufgeführten Probleme gelöst werden durch ein Verfahren zur integrierten onkologischen Eingangsuntersuchung der autologen Tumorantigene und Krebstherapie durch Impfung oder Vakzination mit aus Tumorzellen stammendem Material, welches dadurch gekennzeichnet ist, daß Bestandteile aus Fraktionen, die aus dem bei der an sich bekannten Hormonrezeptoranalyse anfallenden Untersuchungsmaterial erhältlich sind in vitro, immunologisch, biochemisch und/oder molekularbiologisch analysiert werden.

In einer bevorzugten Ausführungsform des erfindungsgemäßen Verfahrens werden die Fraktionen aus dem bei der Hormonrezeptoranalyse anfallenden Tumorzellcytosol und/oder -sediment erhalten. Die Fraktionen beinhalten als Bestandteile Proteine und/oder DNA und/oder RNA, welche analysiert werden. In einer besonders bevorzugten Ausgestaltung des erfindungsgemäßen Verfahrens stammen die Tumorzellen aus Mammakarzinomen, Ovarialtumoren und/oder Gebärmutterkörperschleimhauttumoren. Dabei ist das aus dem Tumorzellcytosol und/oder -sediment erhältliche Protein vorzugsweise tumorrelevantes Antigen. Das erfindungsgemäße Verfahren ermöglicht durch Verwendung der aus dem Tumorzellcytosol und/oder -sediment erhältlichen DNA die Identifizierung von Tumoren mittels Hybridisierung von tumorindizierender DNA eine sichere Diagnose bestimmter Tumoren in vitro.

Die Erfindung wird anhand der folgenden Beispiele des Brust- und/oder des Eierstockkrebses näher erläutert:
1. Beim Mammakarzinom wird weltweit neben der obligatorischen histopathologischen Untersuchung des Primärtumors und des Operationspräparates (Brust mit dem dazugehörigen auxiliaren Fett- und Drüsenpaket) auch die routinemäßige Durchführung der Hormonrezeptoranalytik, sowohl aus dem Primärtumor, als auch aus den eventuell befallenen Lymphknotenmetastasen gefordert und durchgeführt. Hierfür etablierte sich ein logistisches System zwischen der operierenden Klinik, dem pathologischen Institut und dem Hormonrezeptorlabor.
   Bei dieser, zwischenzeitlich zur Routine gewordenen Zusatzuntersuchung, wird das Tumormaterial gekühlt zum Pathologen transportiert. Dort werden die für die Hormonrezeptoranalytik benötigten, bzw. zur Verfügung stehenden Tumormengen tiefgefroren und vorzugsweise unter -21°C zwischengelagert. Der Weitertransport zum Hormonrezeptorlabor erfolgt dann in der Regel unter Trockeneis. Zumindest die Bestimmung und Austitrierung der Estrogen- und Progesteronrezeptorkonzentration erfolgt überall obligatorisch. Das Vorhandensein von Hormonrezeptoren ist die Voraussetzung für die hormonelle Empfindlichkeit der Tumorzellen. Eine hormonelle Therapie ist nur im positiven Fall sinnvoll. Für die Hormonrezeptoranalytik wird das Tumormaterial in Europa aufgrund eines internationalen Konsens (EORTC-Methode) einheitlich vorbereitet und aufgearbeitet. Das Tumorgewebe wird feingeschnitten und mit Hilfe von flüssigem Stickstoff auf ca. -170°C abgekühlt und wird in diesem Zustand mit einem Hammer und in einem speziellen Apparat, in dem sogenannten Dismembrator, pulverisiert. Der Dismembrator besteht aus einer Stahlkugel in einer Teflonkapsel, die vorher ebenfalls auf ca. -170°C abgekühlt wird. Das pulverisierte Tumorgewebe wird anschließend, stets im gekühlten Zustand zwischen 0 und 4°C mit einem Puffergemisch extrahiert. Nichtlösbare Tumorbestandteile werden in einer präparativen Ultrazentrifuge 45-60 Minuten lang bei 105.000 g abgetrennt. Das so gewonnene Tumorsediment wird bis heute weltweit unbenutzt weggeschmissen, zumindest wird dieses Tumorsediment nicht für tumorimmunbiologische Untersuchungen in-vitro oder am Patienten eingesetzt. Der Ultrazentrifugenüberstand bildet das Tumorcytosol. Die Gesamteiweißkonzentration des Tumorcytosols beträgt in der Regel 1,0 bis 10,0 mg/ml. Im Rahmen der Hormonrezeptoranalytik wird zusätzlich die Humanalbuminkonzentration bestimmt und der Gesamteiweißwert des Cytosols wird um die Humanalbuminkonzentration gekürzt. Der so ermittelte Eiweißwert bildet den korrigierten Proteinwert des Tumorcytosols und wird zur Berechnung der Hormonrezeptorkonzentration in Femtomol/mg Tumorprotein herangezogen. Die Albuminkonzentration im Cytosol gibt Aufschluß über die Blutbeimengungen, da Humanalbumin nur in der Leber gebildet wird.
2. Die erfinderische parallele Verwendung der Cytosolreste aus der Hormonrezeptoranalytik und der Einsatz des nicht benutzten Tumorsedimentes hat eine sehr wichtige innovative immunbiologische Relevanz. Hierbei, bei dem kombinierten getrennten Einsatz von autologem Antigen aus dem Tumorcytosol und aus dem Tumorsediment handelt es sich immunbiologisch um vollwertige, native Antigene, die zusammen das gesamte antigene Spektrum der Tumorzellen beinhalten. Im Cytosol befinden sich die cytoplasmatischen Antigene, die im Zellinneren gebildet und zunächst dort zu finden sind. Bei der schonenden, auschließlich mechanischen Präparationstechnik, die stets im gekühlten Zustand stattfindet, gehen nur minimale Anteile der Kernantigene und die zellmembranständigen Antigene in lösbare Form über. Reagiert das so aufgearbeitete Cytosol in einem Testsystem positiv, so ist mit hoher Wahrscheinlichkeit anzunehmen, daß diese Antigene Neoantigene sind, wie z.B. Genprodukte von Krebsviren.
   Bei Krebsviren, wie z.B. bei verschiedenen Subtypen der Papillomviren, die 80% des Gebärmutterhalskrebses mitverursachen, wird das Virusgenom komplett oder zum Teil in das Genom der Tumorzelle integriert. Komplette Krebsviren werden in den seltensten Fällen gebildet. In der Regel werden nur Teilabschnitte des Virusgenoms translatiert, d.h. nur einzelne virale Genprodukte werden in den Tumorzellen exprimiert, die dann als Antigene für die Abwehr des Tumorträgers fungieren.
   Der Anmelder dieser Erfindung ist Inhaber des erteilten europäischen Patentes für die Cytosol-CEA-Bestimmung. Durch die standardisierte Cytosol-CEA-Bestimmung können Mammakarzinome bereits alleine, oder in Kombination mit anderen Untersuchungstechniken, einfach in zwei Biotypen Biotyp-A und Biotyp-B differenziert werden. Biotyp-A wird mit hoher Wahrscheinlichkeit durch ein Retrovirus mitverursacht, da Biotyp-A regelmäßig kreuzreagiert mit gp-55, mit dem Hüllprotein des Maus-Mammatumorivrus, bzw. mit dem Hüllprotein verwandter Retroviren und diese Form des Brustkrebses weist gehäuft Reversetranskriptase Aktivität in Tumorextrakten auf.
   Mit Hilfe der indirekten Laserimmunofluoreszenz bei vitalisolierten Mammakarzinomzellen Biotyp-A kann belegt werden, daß diese cytoplasmatischen Neoantigene in die Zellmembran hineinprozessiert, bzw. durch die Zellmembran durchgeschleust werden können.
   Das Antigen aus dem aufgearbeiteten Tumorsediment hat eine immunbiologische Äquivalenz zu der Zellmembran der Tumorzellen. Das Tumorsediment besteht zwar zum größten Teil aus Gewebsfasern, Zellkerntrümmern, nichtlösbaren Anteilen der Zellorganellen, wie z.B. Mitochondrien, beinhalten jedoch auch die feinsten Partikel der zerrissenen Tumorzellmembran. Dieses Zellmembrankompartiment läßt sich in einem weiteren Schritt auf sehr elegante Weise weiter anreichern, wie es noch dargestellt wird.
   Reagiert eine Antigenpräparation aus dem Tumorsediment nicht in einem immunbiologischen Testsystem, so ist mit hoher Wahrscheinlichkeit davon auszugehen, daß in der Tumorzellmembran keine Antigenität vorhanden ist und immunmodulierende, immuntherapeutische Maßnahmen nicht sinnvoll sind, bzw. nur auf der niedrigsten Ebene der Abwehr, auf der Ebene der Makrophagen- und Natural-Killer-Zellen-Stimulatoren therapeutische Maßnahmen sinnvoll sind. Es ist nämlich ein schulmedizinisches Dogma, daß jede Tumorzelle, wie überaltete somatische Zellen, von den Natural-Killer-Zellen als solche erkannt und angegriffen werden würden.
3. Tumorantigen, aus dem Tumorcytosol und Tumorsediment präpariert, kann in verschiedenen Testsystemen eingesetzt werden, qualitativ abhängig davon, auf welcher Ebene der Immunantwort eine Reaktion bejaht oder verneint werden kann. Medikamentös-therapeutische Maßnahmen werden sinnvollerweise dann gezielt in Abhängigkeit von der Qualität und Stärke der Immunabwehrreaktion durchgeführt.
   In der relevanten Weltliteratur hat beim Brustkrebs aus klinisch-prognostischer Sicht der Leukozyten-Makrophagen-Inhibitionstest (LMI/MIF) an Bedeutung erlangt. Dem Leukozytenadhärenzinhibitionstest (LAI) kann diese Bedeutung nicht zugeordnet werden. Der LAI ist mehr aus der Sicht der immunologischen Brustkrebsfrühdiagnostik/Risikogruppenbildung von Interesse. So liegt es auf der Hand, daß innovative und erfinderische Anwendung von Antigenpräparationen aus dem Tumorcytosol und Tumorsediment zuerst, beispielhaft, auf dieses Testsystem, auf den LMI-Test adaptiert werden sollte.
4. Es wurden diverse Bemühungen unternommen, Antigenpräparationen im LMI-Test einzusetzen. Der Test wird in der Regel mit Tumorgefrierschnitten durchgeführt. Hierzu werden 1 cm² 20 Mikron dicke Tumorschnitte auf einem Deckglas in der Kulturkammer den isolierten Abwehrzellen zur Antigenerkennung angeboten. Um hiervon abzukommen, wurden meistens lösbare Tumorantigene präpariert, wie z.B. 3M KCL. Extrakte, und auch erfolgreich im Testsystem eigesetzt. Zwar gab es Autoren, die sogar formalinfixierte ganze Tumorzellen im LMI-Test als Antigen eingesetzt haben, aber es ist bis heute kein Verfahren beschrieben, das aus dem gleichen Tumorstück parallel, in immunbiologisch relevanter Form, sowohl lösbares als auch nichtlösbares Tumorantigen, mit dem voll erfaßten Antigensprektrum in rationeller Form präparierte und zur Erfassung der tumorassoziierten Immunreaktion einsetzte.
5. So besteht ein Hauptmerkmal des neuen Verfahrens darin, daß für die Antigenpräparation Reste des Tumorstückes mitverwendet werden, das die Hormonrezeptoranalytik, z.B. beim Brust- und Eierstockkrebs, durchläuft. Durch die modernen Untersuchungstechniken, wie Estrogen- und Progesteronrezeptor-Enzymimmunoassay der Fa. Abbott, werden immer weniger Mengen von Tumorcytosolprotein, max. 1 - 3 mg, benötigt. So stehen die Cytosolreste weitgehendst und das Tumorsediment quantitativ für die Antigenpräparation zur Verfügung. Dieses Vorgehen hat eine erhebliche ökonomische Bedeutung. Bei kleinen Tumoren, die zu diagnostizieren und zu therapieren zu wünschen sind, aus der Sicht der besseren Prognose, steht darüberhinausgehend wegen Mangel an der Tumormasse überhaupt keine andere Alternative offen, als die Weiterverwendung der Cytosolreste und des Tumorsediments, wenn man die zwingend notwendigen immunbiologischen Untersuchungen sicherstellen will.
   Auch bei größeren Tumoren, wo getrenntes Tumorstück aufgearbeitet werden könnte, ist es aus ökonomischen Gründen sinnvoller, die Cytosol- und Tumorsedimentreste zum Antigen weiterzupräparieren. Bereits die getrennte Aufhebung, Einsendung (Verpackung, Trockeneis, Versandpapiere und Transportkosten) eines zweiten Tumorstückes in ein zweites Labor macht Zusatzkosten in Höhe von mindestens 40 bis 70 DM aus, von den Kosten einer zweiten Präparation (Pulverisieren, Extrahieren, Ultrazentrifugieren, usw.) gar nicht zu sprechen.
6. Ein zweites Hauptmerkmal des neuen erfinderischen Verfahrens besteht wiederum darin, daß zunächst ohne jegliche chemische Behandlung des Cytosols und des Tumorsediments gearbeitet wird. Damit bleibt das Tumorantigen in seiner biologisch relevanten Form erhalten. Hier wird z.B. nur auf die Wichtigkeit der tumorzellmembrangebundenen Antigene hingewiesen. Bei diesen Antigenen haben nur diejenigen Molekülabschnitte/Epitope, eine immunbiologische Relevanz, die aus der Zellmembran herausragen und so bei den lebenden Tumorzellen dem Abwehrmechanismus des Tumorträgers zugänglich sind. In diesem Sinne wird bei dem erfinderischen neuen Verfahren zunächst nur mit Hilfe von mechanischen Maßnahmen gearbeitet, d.h. die primären lösbaren und nichtlösbaren Antigenpräparationen hergestellt. Diese Maßnahmen bestehen aus Differential- und/oder Gradientenzentrifugation, bzw. aus Differentialfiltration.
   Das Cytosol wird vorzugsweise gegen eine geeignete Pufferlösung, z.B. 0,15M PBS (Phosphatgepufferte physiologische Kochsalzlösung mit oder ohne Mg⁺⁺ und Ca⁺⁺ -Ionen in physiologischer Konzentration) dialysiert, um die verwendeten Chemikalien der Cytosolpräparation für die Hormonrezeptoranalytik, wie z.B. SH-aktive Gruppen zu eliminieren. Erfolgt die Cytosolpräparation ohne unphysiologische chemische Zusätze, so entfällt der Arbeitsschritt des Dialysierens.
   Vorzugsweise wird das 100- bis 500fache Volumen zum Dialysieren verwendet. Das so präparierte Cytosol wird vorzugsweise durch einen Membranfilter mit einer Maschenweite von 0,2 Mikron gefiltert. Dies entspricht einer Sterilfiltration. Diese Cytosol-Antigenpräparation ist bereits geeignet, außer ex-vivo-in-vitro-Untersuchungen, auch im Hauttest, eingesetzt zu werden. Im LMI-Test wird diese Cytosol-Antigenpräparation vorzugsweise in den Konzentrationen 100-, 20-, 4-, 0,8-Mikrogramm korrigierter Tumorprotein per ml Medium eingesetzt. Als Hauttest und für Vakzinations-/Impfzwecke wird man dieses Cytosolantigen selbstverständlich vorzugsweise weiter präparieren. Flüssige, lösbare Antigene, insbesondere dann, wenn diese ein schwaches Antigen darstellen, sind gelöst nicht sehr geeignet für Hauttests oder zur Immunisierung. Hierzu wird man das Cytosolantigen vorzugsweise in einem weiteren Schritt bzw. Schritten noch aufarbeiten und konditionieren. Dies beinhaltet vorzugsweise die weitere Anreicherung der antigenrelevanten Fraktionen, bzw. die Insolubisierung der Antigenfraktionen, wie z.B. die physikochemische Absorption an Aluminiumhydroxid-Gel in pharmazeutischer Qualität. Hierzu sind nach dem Stand der Technik die verschiedensten Anreicherungsmöglichkeiten und Potentierungsmöglichkeiten offen.
   Sehr praktikabel hat sich beim Mammakarzinom die schonende Hitze-Mikropräzipitation erwiesen. Hierzu wird ein Volumen Tumorcytosol mit zwei Volumen 0.2M Azetatpuffer pH 5.0 versetzt und bei 70°C 15 Minuten inkubiert. Auf diese Weise entstehen feinste Proteinmikropräzipitate als feinste Partikel, die nach Abtrennen und Waschen in PBS sehr gut zu resuspendieren sind, mit weitgehendst erhaltengebliebenem Antigenspektrum. Wurde vorher das Cytosol oder die Cytosolfraktion sterilfiltriert, läßt sich die Proteinpartikelsuspension bei aseptischem Weiterverarbeiten als Antigen für Hauttests oder für Vakzination einsetzen. Bei der Vakzination wird man das Mischen mit weiteren immunologisch aktiven Substanzen, wie z.B. BCG oder Propionbakterien bevorzugen. Im hitzestabilen Überstand verbleibt nur ein kleiner Teil der Antigenität des Cytosols.
   Die nicht chemische, nur mechanische Weiteraufarbeitung des Tumorsediments besteht im ersten weiteren Schritt in der Differentialzentrifugation. Das Tumorsediment wird bei der Cytosolpräparation, nach der Zertrümmerung im Dismembrator, durch Ultrazentrifugieren über 100.000 g mit einer Laufzeit bis zu einer Stunde abgetrennt. Unter diesen Konditionen werden praktisch alle nichtlösbaren, partikularen Tumorbestandteile im Sediment abgetrennt. 1 g Tumor nach Feuchtgewicht ergibt im Durchschnitt 20-25 mg Tumorcytosolprotein in 3-5 ml Pufferextrakt und 200-250 mg Tumorsediment mit einem Feuchtigkeits-/Cytosolrestanteil von ca. 10 bis max. 20%.
   Dieses Tumorsediment wird vorzugsweise zunächst im 5-fachen Volumen PBS resuspendiert. Die Resuspension wird durch rasches Aufziehen und Wiederausspritzen durch eine Kanüle mit entsprechender Weite bewerkstelligt. Durch stufenweises Verwenden einer immer feiner werdenden Injektionskanüle wird die Resuspension schrittweise verfeinert. Hierbei wird das Resuspensionsvolumen unter ständigem Kühlen zwischen 0 bis 4°C auf das 25-fache der Sedimentmenge erhöht. Danach werden die Grobpartikel, wie z.B. Bruchstücke von Kollagenfasern usw., vorzugsweise bei nur 1000-1500 G über 10 Minuten, möglichst in einer Kühlzentrifuge bis max. 4°C, abgetrennt und ggf. mit einem weiteren PBS-Puffervolumen erneut ausgewaschen. Das Sediment wird als Fraktion 0 bezeichnet.
   Der Überstand, bzw. die vereinigten Überstände, Fraktion I, ist eine opaleszente, weißliche Suspension von Feinpartikeln und wird vorzugsweise vor dem weiteren Bearbeiten gegen 50- bis 100fache Volumen PBS dialysiert. Fraktion-I kann ebenfalls aufgrund des Gesamteiweißwertes kalibriert werden. In der Regel ist es jedoch ausreichend sich nach der Verdünnungsstufe des ursprünglichen Tumorsedimentes zu richten und so wird nach der Dialyse mit PBS eine Endverdünnung von 50-fach, bezogen auf die ursprüngliche Ultrazentrifugen-Sedimentmenge, eingestellt.
   Die weiteren Schritte der nur-mechanischen Reinigung besteht aus Differentialfiltration. Hierzu wird die Tumorsedimentsuspension jeweils durch einen Membranfilter mit der Maschenweite 20-10-4-0,8-0,2 Mikron gefiltert. Die Abstufungen können frei gewählt werden. Das jeweilige Filtrat bildet die nächste Fraktion, z.B. durch den 20-Mikron-Membranfilter Fraktion II/20, usw. Durch das Rückspülen des jeweiligen Membranfilters gewinnt man den jeweiligen Membranfilterrückstand, bezeichnet z.B. Fraktion II/20-Rückstand usw.
   Von besonderer Bedeutung ist das 0,2-Mikron-Membranfiltrat, da es die feinste Dispersion aufweist und keimfreifiltriert ist. Im LMI-Test und im Hauttest hat man mit diesem Tumorsedimentfiltrat die besten Ergebnisse erzielt. Dieses 0,2-Mikron-Sedimentfiltrat macht etwa nur 1 bis 4% der Gesamttumorsedimentmenge aus und ergibt aus 1 g Tumorgewebe nach Feuchtgewicht ca. 2,5 bis 10,0 mg Partikelsuspenison in ausverdünntem Zustand. Dieses Sedimentfiltrat eignet sich ohne weitere Bearbeitung nicht nur für immunbiologische ex-vivo-in-vitro-Untersuchungen, sondern auch als Antigen für Hauttest und für Vakzination/Impfung. Vorzugsweise wird jedoch dieses Antigenpräparat für diese Anwendung weiter konditioniert, wie Abtrennen und Resuspendieren in höherer Partikelkonzentration, bzw. Absorbieren an Aluminiumhydroxid-Gel, Mischen mit BCG oder mit einem Propionbakterien-Suspensionspräparat als zusätzlicher Immunmodulator. Selbstverständlich wird man nach dem Prinzip der früher beschriebenen Schachbrett-Titration vorzugsweise vorgehen.
   Das 0,2-Mikron-Sedimentfiltrat, Verdünnungsstufe 50fach, wird im LMI-Test optimal in Konzentrationen von 100-, 20-, 4,0- und 0,8-Mikroliter per Kulturmedium eingesetzt. In der Routine kommt man mit zwei, bis max. drei Antigenkonzentrationen aus, wobei die 20- und 4-Mikroliter-Sedimentfiltrate per ml Kulturmedium von der größten Relevanz sind. Diese sind die günstigsten Erfahrungswerte.
   Berechnungen ergeben, daß in der Antigenpräparation aus dem Tumorsediment, nach diesem Verfahren, nicht verschleppte cytoplasmatische, lösbare Antigenreste reagieren, sondern tatsächlich nicht-lösbare, korpuskuläre Antigene, wie z.B. tumorzellmembranständige Antigenstrukturen.
7. Verwendet man das 0,2-Mikron-Membranfiltrat des Tumorsedimentes als Antigen, so geht gegenüber der Tumorsediment-Fraktion I in erheblichem Umfange, ca. 80-90% der Gesamtantigenmenge verloren. Im LMI-Test hat sich jedoch das 0,2-Mikron-Membranfiltrat als optimale Antigenpräparation erwiesen, da größere Partikel, wie z.B. das 10-Mikron-Membranfiltrat, bereits in erheblichem Umfange die scharfe Randbildung der Migrationshöfe stört und dieser Umstand macht die Auswertung in der Syke-Moore-Kammer praktisch unmöglich.
   Ist die Ausbeute an Antigen, wie z.B. für Vakzinationszwecke über einen längeren Zeitraum, nicht ausreichend, so kann bereits das Tumorsediment-Fraktion I für diesen Zweck voll eingesetzt werden, wenn man die Verarbeitung von Anfang an aseptisch durchführt. In diesem Falle sind Sterilitätskontrollen obligat.
8. Das dritte Hauptmerkmal dieser erfinderisch neuen und innovativen Präparationstechnik besteht darin, daß die zerlegte Antigenität des Tumorcytosols (lösbarer Anteil) und des Tumorsedimentes (nicht-lösbarer Anteil) dem Gesamtantigenspektrum einer Tumorzellsuspension entspricht, als wenn man optimalerweise mit isolierten Tumorzellen arbeiten würde. Hierzu ist notwendig, daß vorzugsweise in den immunbiologischen Testsystemen, die angewendet werden, sowohl präpariertes Cytosolantigen, wie präpariertes Sedimentantigen miteingesetzt wird.
9. Im weiteren eröffnet die erfinderische Verwendung des Tumorsedimentes, in der beschriebenen Weise, den Weg membranständige tumorassoziierte Antigene einfach und eleganterweise, ebenfalls in biologisch intaktem Zustand zu isolieren. Der Erfinder konnte in einer früheren Arbeit zeigen, daß beim Mammakarzinom bereits zum Zeitpunkt der Primärtherapie in 21/28(=75%) der Fälle, an der Tumorzellmembran bereits tumorassoziierte Antikörper des Types IG-A, IG-M und/oder IG-G vorhanden sind. Dem Vorhandensein von tumorassoziierten Antikörpern vom Typ-IG-G an der Tumorzellmembran konnte die beste klinische Prognose zugeordnet werden. Es steht eine hochsignifikante Korrelation zum positiven Reaktionsausfall des LMI-Testes mit Inhibition, der ebenfalls eine bessere klinische Prognose zugeordnet werden kann. So besteht ein hohes Interesse daran, auch die tumorassoziierten, membranständigen Antigenstrukturen aus der Tumorsedimentantigenpräparation weiter anzureichern und die Qualität und die Menge der vorhandenen tumorassoziierten Antikörper zu bestimmen. Dies läßt sich rationell, eleganterweise durch eine einfache, Immunabsorptionstechnik bewerkstelligen. Man nimmt hierzu geeignete, immobilisierte Antikörper, die gegen Humanimmunglobuline gerichtet sind. Die relevanten membranständigen Tumorantigene sind mit den tumorassoziierten Antikörpern ja weitgehendst abgedeckt. So reichert man aus dem Tumorsediment durch die Immunabsorptionschromatographie eben diejenige Feinstpartikeln an, die immunbiologisch die relevanten Strukturen für die humorale Immunantwort an sich tragen. So konnte man mit immobilisierten Anti-Human-H-L-Ketten-Antikörpern von der Ziege gegen menschliche Immunglobuline, auf aktivierte Kieselgur (Produkt der Fa. Boehringer Mannheim GmbH) gekoppelt in einem einzigen Arbeitsschritt, diese immunbiologisch hochrelevanten Antigene, die im LMI-Test und im Hauttest hochaktiv sind, um den Faktor 100 anreichern. Als Berechnungsgrundlage diente der Gesamteiweißgehalt des resuspendierten Tumorsedimentes.
   Wird eine Tumorsedimentfraktion von Cytosol-Resten durch Ausverdünnen und erneutes Abzentrifugieren freigewaschen, so besteht die Möglichkeit, diese prognostisch relevanten tumorassoziierten Antikörper, z.B. mit einem Radioimmunoassay, oder mit Hilfe von Techniken der Enzymimmunoassays qualitativ nachzuweisen und semiquantitativ auszutitrieren, um auch für diese Form der tumorassoziierten Immunantwort ein Kontrollsystem in der Hand zu haben.
10. Selbstverständlich ist das Tumorzellsediment aus der Hormonrezeptoranalytik oder unabhängig davon, z.B. bei anderen Krebsformen, wie beim Dickdarmkrebs, in der gleichen erfinderischen Weise präpariert, ein geeignetes Ausgangsmaterial für das weitere Präparieren von Tumorantigenen herkömmlicher, bekannter Weise, oder mit neuen zukünftigen Technologien, wie Techniken der Molekularbiologie. So eine bekannte alte Technik ist das Verfahren der 3M KCL-Extraktion.
   Oben wurde dargelegt, wie problematisch es beim Mammakarzinom sein kann, inbesondere bei kleinen Tumoren, alle notwendigen onkobiochemischen und tumorimmunbiologischen Eingangsuntersuchungen zur Biotypisierung sicherzustellen. Neben den onkobiochemischen und tumorimmunbiologischen Untersuchungen sind zwischenzeitlich auch molekularbiologische Untersuchungsmethoden hinzugekommen, wie zum Beispiel die Bestimmung des HER-2/neu-Onkogens im Mammakarzinomgewebe (D.J. Slamon et al., "Human Breast Cancer: Correlation of Relapse and Survival with Amplification of the HER-2/neu Oncogene", Science 235, 1987, 177-182). Üblicherweise muß für diese molekularbiologische Untersuchung aus einem getrennten Tumorgewebsstück DNA und/oder m-RNA isoliert werden. Es wird eine Methode beschrieben, die es erlaubt, auch diese und ähnliche molekularbiologischen Untersuchungstechniken in ein integriertes onkologisches Eingangsuntersuchungsprogramm zusammenzufassen.
   Beim Mamma- und zum Beispiel Ovarialkarzinom beinhaltet dieses integrierte onkologische Eingangsuntersuchungsprogramm auch die Hormonrezeptoranalyse, wobei die Hormonrezeptoranalyse das Ausgangsmaterial liefert.
   Bei bestimmten Tumoren, bei denen die Hormonrezeptoranalyse keine klinische Relevanz besitzt, jedoch die Gewinnung von lösbaren und nicht-lösbaren (Zellwandbestandteile) Tumorgewebefraktionen als Antigen für tumorimmunbiologische Eingangs- und Kontrolluntersuchungen und die Bestimmung der Tumormarkerkonzentrationen (zum Beispiel CEA und Ca-19-9) im lösbaren Tumoranteil (Cytosol-Fraktion) von immenser Wichtigkeit für die Klinik sind, wie zum Beispiel bei den kolorektalen Karzinomen, wird man die ergänzenden molekularbiologischen Untersuchungen mit den bereits etablierten Untersuchungstechniken zusammenfassen und vorzugsweise in diese Untersuchungsabläufe integrieren, um möglichst rationell zu arbeiten.
   Das Verfahren wird am Beispiel des Mammakarzinoms mit dem HER-2/neu-Onkogen demonstriert.
   Die Grundlage der Hybridisierunstechnik besteht darin, daß DNA und/oder m-RNA aus dem Gewebe bzw. Tumorgewebe isoliert wird, und zwar solche mit hohem Molekulargewicht. Die isolierte DNA wird mit mindestens einem geeigneten Restriktionsenzym aus jeder Probe gleichmäßig geschnitten, durch Agarose-Gel-Elektrophorese aufgetrennt und mit Hilfe einer copy-DNA-Sonde, die bevorzugt mit P³² Isotopen markiert ist, hybridisiert, nachdem die DNA-Proben aus dem Elektrophoresegel auf einen geeigneten Träger, zum Beispiel eine Nylonmembran, überführt worden sind. Die qualitative und quantitative Auswertung erfolgt durch Autoradiographie und Densitometrie des Filmmaterials. Hierdurch wird festgestellt, ob der gesuchte DNA/Genabschnitt in der Probe vorhanden ist, und wenn ja, mit welcher Kopienzahl. Liegt eine Genamplifikation vor, d.h. man findet zwei oder mehrere Kopien dieses Gens in dem Tumorgewebe, so ist dies von höchster prognostischer Relevanz, wie beispielsweise im Falle des HER-2/neu-Onkogens beim Mammakarzinom.
   Eine weitere Aufgabe der Erfindung besteht darin, die ersten Schritte der Tumorgewebeverarbeitung so zu gestalten, daß das integrierte Vorgehen, in diesem Falle die DNA-Isolierung, sichergestellt werden kann. Diese Voraussetzung erfüllt die Cytosol-Präparationstechnik, die in der EP-A-0 092 223 beschrieben ist. Die dort beschriebene Präparationstechnik arbeitet ausschließlich mit Pufferzusätzen und Chemikalien, die die DNA im nativen Zustand belassen. Die DNA der Probe befindet sich fast quantitativ in der Cytosolfraktion. Die Ultrazentrifugation, 105000 G 45 Min. bzw. unter äquivalenten Bedingungen, macht die Cytosolfraktion de facto partikelfrei. Die DNA für die weitergehenden molekularbiologischen Untersuchungen wird aus dieser Tumorcytosol-Fraktion präpariert.
   Die nachstehend beschriebene Präparationstechnik stellt eine geeignete DNA-Isolierungstechnik für diese Aufgabe dar:
   Für die quantitative HER-2/neu-Onkogen-Hybridisierung benötigt man 12 µg Tumor-DNA. Eine Tumorzelle enthält bei normalem diploiden Chromosomensatz ca. 6 pg DNA pro Zelle. So werden für die Isolierung von 12 µg Tumor-DNA 20x10⁶ Tumorzellen benötigt, bei einer 100% DNA-Ausbeute und reiner Tumorzellsuspension. Dies setzt die Verarbeitung von ca. 20 mg Tumorzellsuspension, bzw. unter Berücksichtigung der histopathologischen Besonderheiten des Mammakarzinoms und die Ausbeuterate bei der DNA-Isolierung, 100 bis 200 mg tumorgewebeäquivalente Tumorcytosolfraktion mit einem durchschnittlichen korrigierten Tumorproteinanteil von 5 mg/ml voraus. 1 g Mammakarzinomgewebe ergibt im Durchschnitt 15 bis 25, im Mittel 20 mg Tumorprotein. So ist es der Standardisierung der DNA-Hybridisierung dienlich, wenn einheitlich soviel präpariertes Tumorcytosol, ausgedrückt in ml, für die DNA-Isolierung weiterverarbeitet wird, das gleichzeitig 5 mg korrigiertes Tumorprotein enthält. Die hieraus isolierte Tumor-DNA reicht in der Regel aus, die Untersuchung (DNA-Hybridisierung) im doppelten Ansatz durchzuführen.

Die Schritte der DNA-Isolierung aus dem präparierten Tumorcytosol (Beispiele):
1. Das Tumorcytosol wird mit TBS (Tris-buffered Saline) auf eine korrigierte Tumorproteinkonzentration von 1,0 mg/l verdünnt, jedoch mindestens mit dem zweifachen Volumen TBS pH 7,4.
2. Danach 10 x Volumen von 0,5 M EDTA (pH 8,0), 100 µg/ml Proteinase K, 0,5% Sarcosyl.
3. Inkubation bei 50°C im Wasserbad 3 h. Periodenweise Mischen/Schütteln der viskosen Flüssigkeit.
4. Dreifache Extraktion der DNA mit gleichem Volumen Phenol. Nach der Zentrifugation liegt das Phenol wegen der hohen Salzkonzentration in der oberen Phase. Die Phenolfraktion soll möglichst exakt an der Flüssigkeitsphase abgetrennt werden. Gegebenenfalls (bei wenig Ausgangsmaterial) Reextraktion der Übergangsphase mit zusätzlichem Phenol und Puffer.
5. Nach der dritten Phenolextraktion Dialyse der DNA gegen mindestens 200 x Volumen der Lösung 50 mM Tris-Cl (pH 8,0), 10 mM EDTA und 10 mM NaCl mit wiederholtem Wechseln der Dialyselösung, bis OD₂₇₀ (Optische Dichte bei 270 nm Wellenlänge) weniger als 0,05 wird.
6. Behandlung der dialysierten Probe mit 100 µg/ml DNase-freier RNase 3 h bei 37°C.
7. Intensive Extraktion der Probe zweimal mit 1/1 Volumen Phenol/Chloroform.
8. Erschöpfende Dialyse der Probe gegen TE (10 mM Tris-Cl, pH 8,0; 1 mM EDTA, ph 8,0).
9. Messen der Konzentration der extrahierten DNA mit an sich bekannten Methoden. Falls notwendig, weitere Reinigung und Konzentration der DNA durch Ethanol- oder Isopropanol-Präzipitation, oder Konzentrierung mit Butanolextraktion in an sich bekannter Weise, oder einfach durch Zentrifugation, wie nachstehend beschrieben.
In einem Cäsiumchlorid-Dichtegradienten (p = 1,70) wird die DNA beispielsweise in einem Beckmann Typ-40 Rotor bei 33000 rpm 60 bis 70 h bei 15°C zentrifugiert. Die viskosen DNA enthaltenden Fraktionen werden aus dem Gradienten isoliert und erschöpfend dialysiert gegen Tris-Puffer, pH 8,0, Zusammensetzung s.o.
10. Durchführung und quantitative Auswertung der DNA-Hybridisierung mit HER-2/neu-Onkogensonde, wie bei D.J. Slamon et al., Science 235, 1987 (177-182) beschrieben.
Vergleich der Ergebnisse der Her-2/neu-Onkogen-Hybridisierung beim Mammakarzinom mit DNA-Isolierung klassisch aus getrenntem Tumorstück versus mit DNA-Isolierung aus Tumorcytosol, gewonnen aus den Cytosolresten der Hormonrezeptoranalyse:

| | Einzelkopie | 2-5 Kop. | 5-20 Kop. | mehr als 20 Kop. |
|---|---|---|---|---|
| Einzelkopie | 15 | 1 | 0 | 0 |
| 2-5 Kopien | 1 | 8 | 1 | 0 |
| 5-20 Kopien | 0 | 2 | 3 | 0 |
| mehr als 20 Kopien | 0 | 0 | 0 | 2 |

- horizontal:: Ergebnisse der Hybridisierung mit DNA aus getrenntem Tumorstück
- vertikal:: Ergebnisse der Hybridisierung mit DNA aus Cytosol
Die EP-A-0 092 223 beschreibt ein Konzept, wie im voraus, bereits zum Zeitpunkt der Primärtherapie, diejenigen Mammakarzinompatienten definiert werden können, bei denen man mit den fortlaufenden Serum-CEA-Bestimmungen mit einem hohen positiven Vorhersagewert (praedictive value) eine Progression/Rezidiv frühzeitig, früher oder zumindest gleichzeitig mit den klinischen und radiologischen Untersuchungen erkennen kann.

Zwischenzeitlich sind mehrere neue Tumormarker etabliert worden, wie TPA, Ca-15-3 und MCA für das Mammakarzinom oder Ca-12-5 für das Ovarialkarzinom und Ca-19-9 für die gastrointestinalen Tumoren. Bei Tumoren wie zum Beispiel Ovarialkarzinom, bei denen in 80 bis 85% der Fälle der neue Tumormarker Ca-12-5 von dem jeweiligen Tumor produziert wird und zum Zeitpunkt der klinischen Diagnose meistens eine bereits relativ große Tumormasse gegeben ist, ist die Ca-12-5-Bestimmung im Tumor, zum Beispiel histochemisch oder in die Hormonrezeptoranalyse integriert, aus dem Cytosol onkobiochemisch, ohne klinisches Interesse, da bereits eine einfache Serum-Ca-15-3-Bestimmung eine Aussage über die Verwendbarkeit des Tumormarkers Ca-12-5 erlaubt.

Beim Mammakarzinom ist die Situation für TPA, Ca-15-3 und MCA grundsätzlich anders. Auch kleine Mammakarzinomen können rezidivieren. Bei kleineren Tumoren wie Mammakarzinomen kann nicht erwartet werden, daß es zum Zeitpunkt der Diagnose/Primärtherapie bereits zu einer Erhöhung des jeweiligen Tumormarkerspiegels kommt, auch dann nicht, wenn vom Tumor der fragliche Tumormarker in ausreichendem Umfang produziert und sezerniert wird. So bedarf es umfangreicher Untersuchungen prospektiv oder retrospektiv gut dokumentierter Serum- und Tumor(Cytosol)-Archive, um eine Aussage darüber treffen zu können, inwieweit durch einen prädiktiven Test auf histochemischer Basis oder die quantitative Bestimmung dieses Tumormarkers in einer Fraktion des Tumors, zum Beispiel im Cytosol oder in einer Membranfraktion, der neue Tumormarker in seiner Brauchbarkeit oder Verwendbarkeit für Rezidivfrüherkennung, in welchem Umfang auf den einzelnen Patienten bezogen, eingesetzt werden kann bzw. eingesetzt werden sollte.

Die in der EP-A-0 092 223 beschriebene Cytosol-CEA-Bestimmung kann nicht ohne weiteres auf neue Tumormarker übernommen werden. So stellten sich für die Tumormarker TPA einerseits und für MCA Ca-15-3 andererseits beim Mammakarzinom grundlegend neue Erkenntnisse heraus:

TPA gehört als Substanz/Antigen zu der Gruppe der zellulären Stützsubstanzen, zu den sogenannten Cytoskelett-Proteinen. Erhöhte Serum-TPA-Werte treten auch nicht nur im Falle eines Tumorwachstums/Rezidives auf, sondern auch zum Beispiel bei der Regeneration von Darmschleimhaut nach einer Durchfallerkrankung. Trotzdem stellte sich beim Mammakarzinom für TPA heraus, daß die standardisierte TPA-Bestimmung im Tumorcytosol ein zusätzliches Aggressivitätskriterium darstellt. Je höher die Konzentration von TPA im Tumorcytosol ist, um so aggressiver ist der Tumor. Standardisiert man die Cytosol-TPA-Bestimmung auf die Reagenzien der Firma Sangtec (Schweden) mit der IRMA-Technik, so liegt der Mittelwert bei 155 Unit/mg korrigiertes Tumorprotein, und der Median beträgt 120 Unit/mg korrigiertes Tumorprotein, mit einer Variationsbreite der TPA-Konzentrationen von 22 bis 1500 Unit/mg korrigiertes Tumorprotein. Damit stellt die TPA-Cytosolkonzentration einen wichtigen Prognosefaktor dar.

Andererseits stellte sich für die Ca-15-3- und MCA-Bestimmung im Cytosol beim Mammakarzinom heraus, daß eine klinische Relevanz nur im negativen Sinne gegeben ist, d.h. durch die Cytosol-Ca-15-3- und MCA-Bestimmungen nur diejenigen Mammakarzinomfälle identifiziert werden können, die mit gewisser Wahrscheinlichkeit (negativer Vorhersagewert, negative praedictive value, gleich oder kleiner als 0,7) im Falle eines Rezidives/Progression keine pathologisch erhöhten Werte zeigen bzw. deren pathologisch erhöhte Werte erst deutlich später auftreten, als ein Rezidiv klinisch und/oder radiologisch erkannt werden kann. Eine positive Vorhersage für die beiden Tumormarker Ca-15-3 und MCA beim Mammakarzinom ist nicht möglich, d.h. diejenigen Mammakarzinomfälle im voraus festzulegen, bei denen für die Rezidivfrüherkennung die fortlaufenden Serum-Ca-15-3- und MCA-Bestimmungen geboten waren. Diese Feststellung ist vom Prinzip der Cytosol-CEA-Bestimmung grundsätzlich abweichend.

Auch die obigen Aussagen, Cytosol-TPA-Konzentration als Aggressivitätskriterium beim Mammakarzinom und Cytosol-Ca-15-3- und -MCA-Konzentration als Ausschlußkriterium für den Einsatz dieser Tumormarker in der Mammakarzinomnachsorge, sind nur möglich, wenn die Cytosol-TPA-, Ca-15-3- und MCA-Konzentration standardisiert, d.h. von Labor zu Labor einheitlich, möglichst mit den Reagenzien des gleichen Herstellers bestimmt und die Reagenzien dieses Herstellers durch einen Cytosol-Standard stets kontrolliert und geeicht werden.

Die Interpretation der Werte der standardisierten Cytosol-TPA -, Ca-15-3- und -MCA-Bestimmung beim Mammakarzinom ist für den klinischen Einsatz grundsätzlich anders als bei der beschriebenen Cytosol-CEA-Bestimmung. Diese Erkenntnisse können auch auf weitere bekannte Tumormarker wie Ca-19-9 oder sogar auf zukünftige neue Tumormarker übertragen werden. Ob die Bestimmung eines Tumormarkers im Cytosol sinnvoll oder wertlos ist und wie die Ergebnisse für die Klinik zu interpretieren sind, setzt erstens die Bestimmung dieses Tumormarkers im Cytosol unter standardisierten Bedingungen voraus und zweitens ist es zwingend notwendig, an einem größeren Patientenkollektiv und über einen längeren Zeitraum Erfahrungen zu sammeln, inwieweit aus diesen Werten für die Prognose und für die Brauchbarkeit des jeweiligen Tumormarkers als Serumspiegelbestimmung Grenzwerte/Diskriminierungswerte abgeleitet werden können oder nicht.

So konnte für den Tumormarker Ca-15-3 beim Mammakarzinom nach dem oben beschriebenen Prinzip im Tumorcytosol der Grenzwert 10,0 mE Ca-15-3/mg korrigiertes Tumorprotein ermittelt werden, von welchem Punkt/Grenzwert an der Einsatz von Ca-15-3 als Tumormarkerbestimung in der Nachsorge als Blutuntersuchung kritisch, d.h. unwirtschaftlich wird. Dieser Grenzwert wurde unter standardisierten Bestimmungen für die RIA-Reagenzien der Firma Isotopendienst-West (CIS)/Bundesrepublik Deutschland ermittelt.

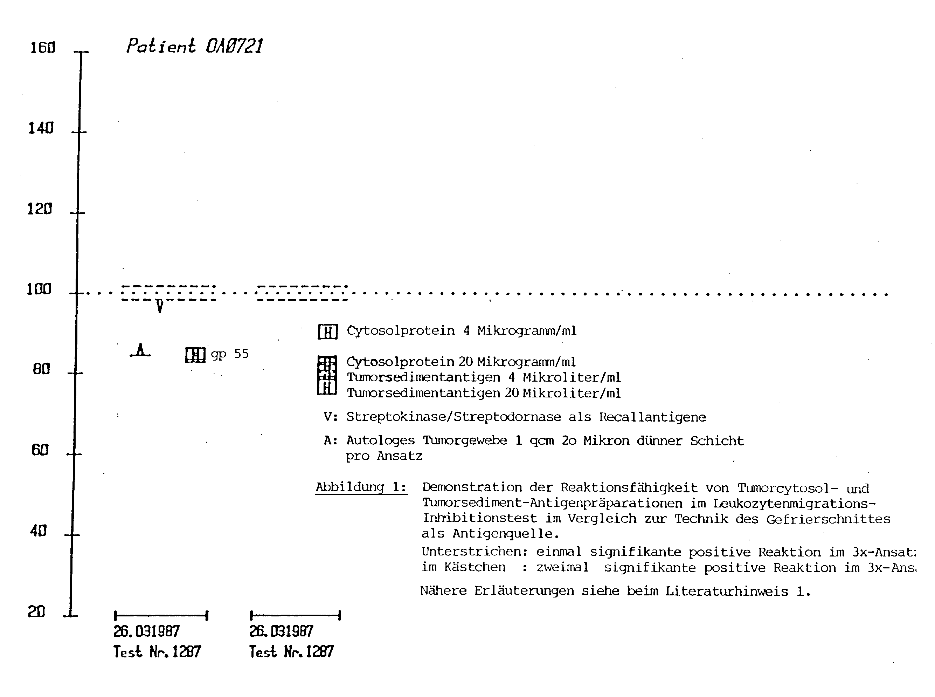

## Patentansprüche

1. Verfahren zur integrierten onkologischen Eingangsuntersuchung der autologen Tumorantigene und Vorbereitung der Krebstherapie durch Impfung oder Vakzination mit aus Tumorzellen stammendem Material, dadurch gekennzeichnet, daß Bestandteile aus Fraktionen, die aus dem bei der an sich bekannten Hormonrezeptoranalyse anfallenden Untersuchungsmaterial erhältlich sind in vitro, immunologisch, biochemisch und/oder molokularbiologisch analysiert werden.

2. Verfahren nach Anspruch 1, dadurch gekennzeichnet, daß die Fraktionen aus dem bei der Hormonrezeptoranalyse anfallenden Tumorzellcytosol und/oder -sediment erhältlich sind.

3. Verfahren nach Anspruch 1 oder 2, dadurch gekennzeichnet, daß die Bestandteile Proteine und/oder DNA und/oder RNA analysiert werden.

4. Verfahren nach einem der Ansprüche 1 bis 3, dadurch gekennzeichnet, daß die Tumorzellen aus Mammakarzinomen, Ovarialtumoren und/oder Gebärmutterkörperschleimhauttumoren stammen.

5. Verfahren nach einem der Ansprüche 1 bis 4, dadurch gekennzeichnet, daß das aus dem Tumorzellcytosol und/oder -sediment erhältliche Protein tumorrelevantes Antigen ist.

6. Verfahren nach einem der Ansprüche 1 bis 5, dadurch gekennzeichnet, daß das Tumorzellcytosol und das -sediment aus der Hormonrezeptoranalyse mechanisch, durch Dialyse, Differential- und/oder Gradientenzentrifugation sowie durch Differentialfiltration zum Antigen präpariert wird.

7. Verfahren nach einem der Ansprüche 1 bis 6 zur Herstellung von autologem Tumorantigen in lösbarer und in nicht lösbarer Fraktion für immunbiologische Untersuchungen, für Hauttest- und für Impf-/Vakzinationszwecke, dadurch gekennzeichnet, daß diese biologisch mit vollem Antigenspektrum präsente Tumorantigenpräparationen in der Weise gewonnen/präpariert werden, daß zunächst der Tumor/die Tumorzellsuspension homogenisiert, vorzugsweise in flüssigem Stickstoff tiefgefroren (mit einem Hammer und mit einem sogenannten Dismembrator), pulverisiert und mit einem geeigneten Puffer extrahiert wird und anschließend die nicht lösbaren Tumoranteile durch Ultrazentrifugation über 10.000 g, vorzugsweise über 100.000 g über 45-60 Minuten, abgetrennt werden und im weiteren die lösbare Tumorantigenfraktion durch Dialyse und/oder durch Sterilfiltration zum Antigeneinsatz vorbereitet wird, währenddessen die nicht lösbare Tumorantigenfraktion, das Tumorsediment, zunächst durch Differential- und/oder Gradientenzentrifugation sowie durch Differentialfiltration zum Antigeneinsatz weitervorbereitet wird.

8. Verfahren nach Anspruch 7 zur weiteren Tumorantigenpräparation/Fraktionierung/Extrahierung und weiteren chemischen Aufarbeitung, dadurch gekennzeichnet, daß das Ausgangsmaterial vorfraktioniert wurde.

9. Verfahren zur Herstellung membranständiger tumorassoziierter Antigene nach Anspruch 7, dadurch gekennzeichnet, daß durch einfache Immunabsorbtionschromatographie aus der nicht-lösbaren Tumorsediment-Antigenfraktion diejenigen Antigenfraktionen abgetrennt werden, die durch natürliche tumorassoziierte Immunglobuline des Patienten selbst bereits abgedeckt, markiert sind.

10. Verfahren nach einem der Ansprüche 1 bis 3, dadurch gekennzeichnet, daß die erhältliche DNA-Fraktion mit komplementärer tumorindizierender DNA hybridisiert wird.

11. Verwendung von in vitro aus der Hormonrezeptoranalyse stammendem Material zur Herstellung von Fraktionen enthaltend autologe Tumorantigene, die zur integrierten onkologischen Eingangsuntersuchung und Tumortherapie geeignet sind.

12. Autologe Tumorantigenpräparationen in lösbarer und in nicht lösbarer Fraktion für immunbiologische Untersuchungen, für Hauttest- und für Impf-/Vakzinationszwecke, dadurch gekennzeichnet, daß diese biologisch mit vollem Antigenspektrum präsente Tumorantigenpräparationen in der Weise gewonnen/präpariert werden, daß zunächst der Tumor/die Tumorzellsuspension homogenisiert, vorzugsweise in flüssigem Stickstoff tiefgefroren (mit einem Hammer und mit einem sogenannten Dismembrator), pulverisiert und mit einem geeigneten Puffer extrahiert wird und anschließend die nicht lösbaren Tumoranteile durch Ultrazentrifugation über 10.000 g, vorzugsweise über 100.000 g über 45-60 Minuten, abgetrennt werden und im weiteren die lösbare Tumorantigenfraktion durch Dialyse und/oder durch Sterilfiltration zum Antigeneinsatz vorbereitet wird, währenddessen die nicht lösbare Tumorantigenfraktion, das Tumorsediment, zunächst durch Differential- und/oder Gradientenzentrifugation sowie durch Differentialfiltration zum Antigeneinsatz weitervorbereitet wird.

## Claims

1. A method for the integrated oncologic initial examination of the autologous tumour antigens and for the preparation of cancer therapy by inoculating or vaccinating tumour cell-derived material, characterized in that components of fractions obtainable from the material of investigation recovered from the per se known hormone receptor analysis are analyzed in vitro by means of immunology, biochemistry and/or molecular biology.

2. The method according to claim 1, characterized in that the fractions can be obtained from the tumour cell cytosol and/or sediment recovered in the hormone receptor analysis.

3. The method according to claim 1 or 2, characterized in that components being proteins and/or DNA and/or RNA are analyzed.

4. The method according to one of claims 1 to 3, characterized in that the tumour cells have been derived from mammal carcinomae, ovarian tumours and/or uterus body endometrium tumours.

5. The method according to one of claims 1 to 4, characterized in that the protein obtainable from tumour cell cytosol and/or sediment is a tumour-relevant antigen.

6. The method according to one of claims 1 to 5, characterized in that the tumour cell cytosol and the sediment from hormone receptor analysis are mechanically prepared by dialysis, differential and/or gradient centrifugation and by differential filtration to form the antigen.

7. The method according to one of claims 1 to 6 for preparing an autologous tumour antigen in a dissoluble or non-dissoluble fraction for immunobiological investigations, for skin test purposes and for inoculation/vaccination purposes, characterized in that these tumour antigen preparations which are biologically present with the full antigen spectrum are recovered/prepared in such manner that first the tumour/the tumour cell suspension is homogenized, preferably frozen in liquid nitrogen, powdered (using a hammer and a so-called dismembrator), and extracted using a suitable buffer, and subsequently, the non-dissoluble tumour portions are separated by ultracentrifugation at above 10,000 g, and preferably above 100,000 g for 45 to 60 minutes, and further, the dissoluble tumour antigen fraction is prepared for antigen use by dialysis and/or sterile filtration, while the non-dissoluble tumour antigen fraction, the tumour sediment, first is further prepared for antigen use by differential and/or gradient centrifugation and by differential filtration.

8. The method according to claim 7 for further tumour antigen preparation/fractionation/extraction and further chemical processing, characterized in that the starting material is pre-fractionated.

9. The method for preparing membrane-located tumour-associated antigens according to claim 7, characterized in that from the non-dissoluble tumour sediment antigen fraction by simple immunoadsorption chromatography those antigen fractions are removed which are already covered, labelled by natural tumour-associated immunoglobulins of the patient himself.

10. The method according to anyone of claims 1 to 3, characterized in that the obtainable DNA fraction is hybridized with complementary tumour-indicating DNA.

11. In vitro use of material derived from hormone receptor analysis and containing autologous tumour antigens for the preparation of fractions suitable for integrated oncologic initial examination and tumour therapy.

12. Autologous tumour antigen preparations in a dissoluble and in a non-dissoluble fraction for immunobiological investigations, for skin test purposes and for inoculation/vaccination purposes, characterized in that these tumour antigen preparations which are biologically present with the full antigen spectrum are recovered/prepared in such manner that first the tumour/the tumour cell suspension is homogenized, preferably frozen in liquid nitrogen, powdered (using a hammer and a so-called dismembrator), and extracted using a suitable buffer, and subsequently, the non-dissoluble tumour portions are separated by ultracentrifugation at above 10,000 g, and preferably above 100,000 g for 45 to 60 minutes, and further, the dissoluble tumour antigen fraction is prepared for antigen use by dialysis and/or sterile filtration, while the non-dissoluble tumour antigen fraction, the tumour sediment, first is further prepared for antigen use by differential and/or gradient centrifugation and by differential filtration.

## Revendications

1. Procédé d'examen oncologique intégré des antigènes tumoraux autologues et de préparation à la thérapie anticancéreuse par inoculation ou vaccination avec une substance provenant de cellules tumorales, caractérisé en ce que des constituants provenant de fractions qu'on peut obtenir in vitro à partir de la substance d'examen obtenu lors de l'analyse connue de récepteurs d'hormones sont analysés par --------------- l'immunologie, ------- biochimie et/ou ----- biologie moléculaire.

2. Procédé selon la revendication 1, caractérisé en ce qu'on peut obtenir les fractions à partir du cytosol et/ou du sédiment de cellules tumorales, obtenu lors de l'analyse de récepteurs d'hormones.

3. Procédé selon la revendication 1 ou 2, caractérisé en ce qu'on analyse les constituants protéines et/ou ADN et/ou ARN.

4. Procédé selon l'une des revendications 1 à 3, caractérisé en ce que les cellules tumorales proviennent de carcinomes du sein, de tumeurs ovariennes et/ou de tumeurs de la muqueuse du corps de l'utérus.

5. Procédé selon l'une des revendications 1 à 4, caractérisé en ce que la protéine ou'on peut obtenir à partir du cytosol et/ou du sédiment de cellules tumorales est un antigène associé à une tumeur.

6. Procédé selon l'une des revendications 1 à 5, caractérisé en ce que le cytosol et/ou le sédiment de cellules tumorales obtenu par analyse de récepteurs d'hormones est transformé en antigène mécaniquement, par dialyse, par centrifugation différentielle et/ou sur gradient, ainsi que par filtration différentielle.

7. Procédé selon l'une des revendications 1 à 6 pour la préparation d'un antigène tumoral autologue sous la forme d'une fraction soluble et d'une fraction non soluble pour des examens immunobiologiques, à des fins de tests cutanés et d'inoculation/vaccination, caractérisé en ce que, pour obtenir/préparer ces préparations d'antigènes tumoraux présents biologiquement avec un spectre antigénique complet, d'abord on homogénéise la tumeur/la suspension de cellules tumorales, de préférence on la congèle dans de l'azote liquide, on la pulvérise (avec un marteau et un appareil dit "dismembrator" ) , on l'extrait avec un tampon approprié et ensuite, on sépare les parties tumorales non solubles, par ultracentrifugation à plus de 10 000g, de préférence plus de 100 000 g, pendant 45-60 minutes, et ensuite, on transforme la fraction soluble d'antigène tumoral en une préparation antigénique, par dialyse et/ou par filtration stérile, tandis qu'on continue la transformation de la fraction non soluble d'antigène tumoral, c'est-à-dire le sédiment tumoral, en une préparation antigénique, d'abord par centrifugation différentielle et/ou sur gradient, puis par filtration différentielle.

8. Procédé selon la revendication 7 pour la préparation, le fractionnement et l'extraction complémentaires d un antigène tumoral et son retraitement chimique complémentaire, caractérisé en ce qu'on fractionne préalablement le matériau de départ.

9. Procédé de préparation d'antigènes associés à des tumeurs à membrane stable ----- selon la revendication 7, caractérisé en ce qu'on sépare, par simple chromatographie d'immuno-absorption de la fraction antigénique non soluble du sédiment tumoral, les fractions antigéniques qui, déjà recouvertes par des immunoglobulines naturelles associées à des tumeurs, du patient lui-même, sont marquées.

10. Procédé selon l'une des revendications 1 à 3, caractérisé en ce que la fraction d'ADN pouvant être obtenue est hybridée avec un ADN complémentaire indiquant une tumeur.

11. Utilisation d'une substance provenant de l'analyse in vitro de récepteurs d'hormones pour la préparation de fractions contenant des antigènes tumoraux autologues, qui conviennent pour l'examen oncologique de départ et la thérapie antitumorale intégrés.

12. Préparations autologues d'antigènes tumoraux sous la forme d'une fraction soluble et d'une fraction non soluble pour des examens immunobiologiques, à des fins de tests cutanés et à des fins d'inoculation/vaccination, caractérisées en ce que, pour obtenir/préparer ces préparations d'antigènes tumoraux présents biologiquement avec un spectre antigénique complet, d'abord on homogénéise la tumeur/la suspension de cellules tumorales, de préférence on la congèle dans de l'azote liquide, on la pulvérise (avec un marteau et un appareil dit "dismembrator" ), on l'extrait avec un tampon approprié et ensuite, on sépare les parties de tumeurs non solubles, par ultracentrifugation à plus de 10 000g, de préférence plus de 100 000g, pendant 45-60 minutes, et ensuite, on transforme la fraction soluble d'antigène tumoral en une préparation antigénique, par dialyse et/ou par filtration stérile, tandis qu'on continue la transformation de la fraction non soluble d'antigène tumoral, c'est-à-dire le sédiment tumoral, en une préparation antigénique, d'abord par centrifugation différentielle et/ou sur gradient, puis par filtration différentielle.
